# EUROPEAN PATENT APPLICATION

(11) **EP 4 661 023 A1**
(43) Date of publication of application: **10.12.2025**
(21) Application number: 25179934.2
(22) Date of filing: 30.05.2025
(51) Int. Cl.: G16H 40/67, G16H 10/40, G16H 10/20, G16H 10/60

(54) **SYSTEMS AND METHODS FOR MAINTAINING DATA INTEGRITY IN A HEALTH ANALYSIS PLATFORM BY ASSESSING AND MODIFYING PHYSIOLOGICAL MEASUREMENTS BASED ON FILTERED HEALTHCARE DATA**

(30) Priority: 03.06.2024 US 202418732403
(71) Applicant: Medidata Solutions, Inc., New York, NY 10014 (US)
(72) Inventor: KHO, Soon Jye, New York, 10014 (US); STRAIT, Caleb, New York, 10014 (US); KODURU, Bhargav, New York, 10014 (US); BURKE, Kelly, New York, 10014 (US)
(74) Representative: Fish & Richardson P.C.

(57) **Abstract**

Systems and methods for maintaining data integrity in a computerized health analysis platform are disclosed. For instance, a method includes (i) filtering existing healthcare data by first determining or extracting a first subset of data of data sets, such that the first subset is focused on common health-related attribute(s), (ii) generating a reference measurement range from the extracted first subset, and (iii) determining, based on the reference measurement range, measurement unit for lab test data that lack measurement unit or exhibit mislabeling error. For instance, the first subset of data sets represents measurements of physiological parameter(s) of entities. For instance, the lab test data are different from the first subset or the existing healthcare data that is used to determine the first subset. After the measurement unit is determined, a data structure representing the measurement unit is generated and stored.

## Description

### TECHNICAL FIELD

This description generally relates to systems and methods for maintaining data integrity in a health analysis platform by assessing and modifying physiological measurements for filtered healthcare data.

### BACKGROUND

In general, a user's health can be assessed by measuring one or more physiological characteristics of the user and comparing the measured physiological characteristics to a health reference. For instance, the health reference can correspond to, or be derived from, existing healthcare data, including historical data, prior clinical trial data, real-time data, and other existing healthcare data. Accordingly, having accurately measured existing healthcare data can improve the quality of the assessment.

### SUMMARY

Implementations according to this disclosure includes a system for maintaining data integrity in a computerized health analysis platform. The system includes at least one processor and a memory subsystem communicatively coupled to the at least one processor. The memory subsystem stores instructions which, when executed by the at least one processor, cause the at least one processor to perform operations including (i) accessing one or more first data structure including a plurality of first data sets regarding a plurality of entities, (ii) determining a first subset of the first data sets based on one or more health-related attributes of the plurality of entities, (iii) generating, in one or more measurement units, a reference measurement range of the one or more physiological parameters of plurality of entities of the first subset of the first data sets, (iv) accessing one or more second data structures including one or more second data sets regarding one or more target entities, (v) determining, based on the reference measurement range, a measurement unit for the one or more measurements that lacks the measurement unit or exhibits the mislabeling error regarding the measurement unit, (vi) generating a third data structure representing the measurement unit, and (vii) storing the third data structure in a hardware storage device. Each of the first data sets represents measurements of one or more physiological parameters of the plurality of entities and one or more health-related attributes of the plurality of entities, where a machine transmitting the data structures determines that the system accessing them is authorized to access them. The one or more second data sets represent one or more measurements of the one or more physiological parameters of the one or more target entities, where the one or more target entities exhibit the one or more health-related attributes. The one or more measurements of the one or more target entities lacks a measurement unit or exhibits a mislabeling error regarding the measurement unit.

Implementations according to this disclosure includes a method for maintaining data integrity in a computerized health analysis platform. The method includes (i) accessing, by an electronic device, one or more first data structure including a plurality of first data sets regarding a plurality of entities, (ii) determining, by the electronic device, a first subset of the first data sets based on the one or more health-related attributes of the plurality of entities, (iii) generating, in one or more measurement units and by the electronic device, a reference measurement range of the one or more physiological parameters of plurality of entities of the first subset of the first data sets, (iv) accessing, by the electronic device, one or more second data structures including one or more second data sets regarding one or more target entities, (v) determining, based on the reference measurement range and by the electronic device, a measurement unit for the one or more measurements that lacks the measurement unit or exhibits the mislabeling error regarding the measurement unit, (vi) generating, by the electronic device, a third data structure representing the measurement unit, and (vii) storing, by the electronic device the third data structure in a hardware storage device. Each of the first data sets represents measurements of one or more physiological parameters of the plurality of entities and one or more health-related attributes of the plurality of entities, where a machine transmitting the data structures determines that the system accessing them is authorized to access them. The one or more second data sets represent one or more measurements of the one or more physiological parameters of the one or more target entities, where the one or more target entities exhibit the one or more health-related attributes. The one or more measurements of the one or more target entities lacks a measurement unit or exhibits a mislabeling error regarding the measurement unit.

Other embodiments of this aspect include corresponding computer systems, apparatus, and computer programs recorded on one or more computer storage devices, each configured to perform the actions or operations described herein. A system of one or more computers can be configured to perform particular actions by virtue of having software, firmware, hardware, or a combination of them installed on the system that in operation causes or cause the system to perform the actions. One or more computer programs can be configured to perform particular actions by virtue of including instructions that, when executed by a data processing apparatus, cause the apparatus to perform the actions.

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1 is a diagram of an example data integrity maintenance system.
FIG. 2 is an example implementation of software or algorithm that is utilized by an electronic device.
FIG. 3 is a flow chart diagram of an example process for determining a measurement unit for lab test data.
FIG. 4 is a flow chart diagram of an example process for determining a measurement unit for lab test data and modifying the lab test data.
FIG. 5 is a diagram of an example computer system.
FIG. 6 is a frequency graph that illustrates a relationship between a frequency of physiological measurements and measurement units.

Like reference numbers and designations in the various drawings indicate like elements.

### DETAILED DESCRIPTION

Accurate lab test data is important in various aspects of healthcare. For instance, in clinical settings, accurate lab test results are critical for diagnosing and monitoring diseases, as they provide critical information about a patient's health status. For instance, blood test data can include important physiological parameter(s) that indicate conditions or indications such as diabetes, cardiovascular diseases, and infections. Accurate lab test data is also important in clinical trials, where it serves as a reference data to evaluate the efficacy and safety of treatments. Further, in research settings, accurate lab test data forms a basis for achieving accurate understanding, correct diagnosis, and advancing medical research. Without accuracy, lab test data can lead to misdiagnoses, ineffective treatments, and misleading research outcomes, which in turn, can compromise patient safety and block medical advancements.

However, lab test data can exhibit data inconsistencies or errors. For instance, lab test data including measurements (e.g., for physiological parameters) can lack measurement unit or exhibit mislabeling error.

For instance, inconsistencies can frequently arise due to various reasons including variations in method, specimen, measurement unit, in-vitro diagnostic devices, differing data entry standards, human errors, sensor inaccuracies, data format discrepancies, and more.

In particular, when measurement units for data entries are missing or mislabeled (e.g., measurement unit is incorrectly labeled or incorrectly documented), this can lead to significant errors in data interpretation and analysis. For example, if a lab report mistakenly labels cholesterol levels in milligrams per deciliter (mg/dL) as grams per liter (g/L), it could lead to a misunderstanding of the patient's health status and potentially to inappropriate treatment decisions.

Accordingly, when healthcare assessments, treatment decisions, or medical research rely on such lab test data that lack measurement unit or exhibit mislabeling error, it can lead to misdiagnoses, ineffective treatments, and misleading research outcomes, which in turn, can lead to compromised patient safety and hindrance on medical advancements.

Implementations according to this disclosure address data issues described above by at least [1] filtering the existing healthcare data (e.g., historical data, prior clinical trial data, real-time data, and other existing healthcare data) by first determining or extracting a first subset of data, such that the first subset of data is focused on common health-related attribute(s), [2] generating a reference measurement range from the extracted first subset of data, and [3] determining, based on the reference measurement range, measurement unit for lab test data that lack measurement unit or exhibit mislabeling error.

For instance, a data integrity maintenance system can filter such existing healthcare data by at least first determining or extracting the first subset of data based on health-related attribute(s). As an example, the first subset of time-series data can be determined or extracted based on one or more health-related attributes which matches those of the lab test data subject to evaluation (e.g., for measurement data accuracy). The health-related attributes can include disease indication, a medical condition other than the disease indication, same medication usage, same medical treatment, a gender, or the like. This determination or extraction of the first subset of time-series data can be advantageous, as the scope of physiological measurements data can be tailored based on commonalities in health-related attribute(s). Accordingly, by generating a reference measurement range based on these commonalities in health-related attribute(s) between the existing healthcare data and the lab data, more accurate evaluation can be conducted on the lab data based on such reference measurement range. For instance, the reference measurement range can be used for determining whether the lab test data lack measurement unit or exhibit mislabeling error and determining the correct measurement unit for the corresponding lab test data.

After the first subset of data is determined or extracted based on the health-related attribute(s), the reference measurement range can be generated. For instance, generating the reference measurement range includes [1] comparing physiological parameters of the first subset of data with each other and [2] converting different measurement units of the physiological parameters of the first subset to one or more measurement units. By filtering the first subset of data to have the reference measurement range in one or more measurement units, it ensures data uniformity and data comparability. For instance, when reference measurement ranges are generated in one or more single units (e.g., multiple reference ranges of cholesterol levels in mg/dL, g/L, etc.), such reference measurement ranges not only ensure data uniformity, but also lead to simplified calculations and error reductions (e.g., when comparing the reference measurement range to different data or lab test data).

After the reference measurement range is generated, measurement unit(s) for lab test data that lack measurement unit or exhibit mislabeling error can be determined based on the reference measurement range (that is tailored based on health-related attribute(s)). Moreover, prior to determining the measurement unit, determination of whether lab data lacks measurement unit or exhibits the mislabeling error can be made. For instance, based on a comparison of measurement values of lab data that shares common health-related attribute(s) with the reference measurement range, it can be determined whether lab test data lack the measurement unit or exhibit the mislabeling error. Once the measurement unit is determined for the corresponding lab test data that lack measurement unit or exhibits labeling error, an indication or notice can be generated on the display of user interface, as well as a frequency graph that represents a relationship between a frequency of measurements and measurement units.

Further, data quality of the lab data can be improved by preventing processing of incomplete or erroneous lab data. The data quality of the lab data can be improved by incorporating the determined measurement unit(s) into the missing measurement unit of the corresponding lab data or replacing the respective measurement unit of the corresponding lab data exhibiting labeling errors with the determined measurement unit(s).

Further, based on improvement of the data quality, the accuracy of lab data can lead to reliable health assessments, effective treatment decisions, and facilitations of medical research. For instance, such accurate lab data can be used as reference data for clinical trials to evaluate the efficacy and safety of treatments, for current health monitoring and diagnosis in assessing patient conditions, and for research purposes.

Further, the embodiments described herein can also reduce the amount computer resources that are consumed while processing healthcare data. For instance, when generating health assessments, a computer system that encounters low quality data may generate results having errors and/or inconsistencies that are not suitable for use. Thus, the computer system may reprocess the data multiple times (e.g., based on manual feedback from a user) until a satisfactory result is achieved. These repeated operations can increase the amount of computation resources (e.g., CPU utilization), memory resources, storage resources, etc.) that are consumed during the health assessment process. The embodiments described herein can be used to automatically identify and correct labeling errors and/or inconsistencies, thereby [1] reducing the likelihood that healthcare data is reprocessed due to low quality and [2] reducing the consumption of computer resources.

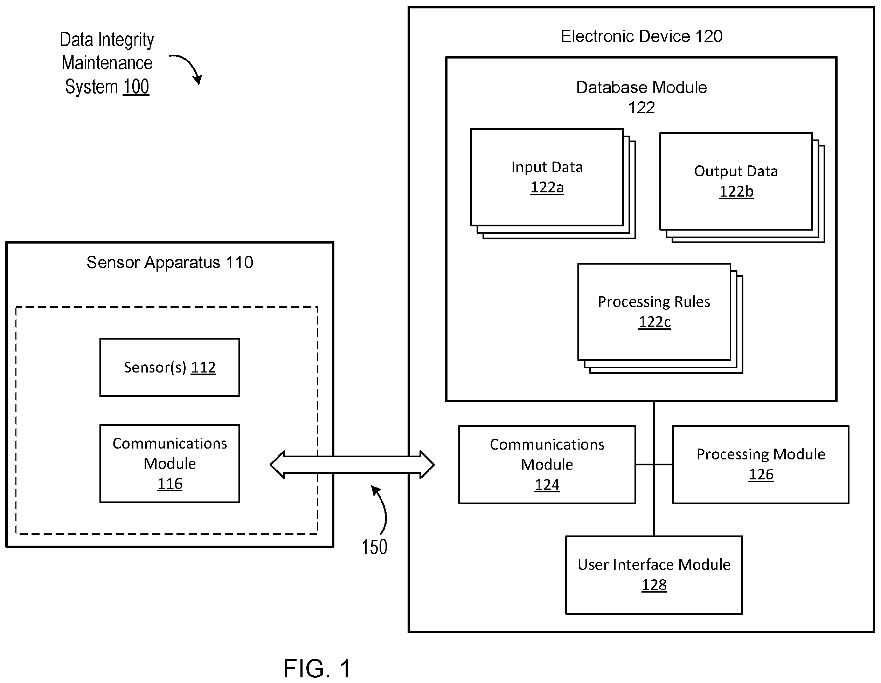

FIG. 1 shows an example data integrity maintenance system 100. In particular, the data integrity maintenance system 100 can [1] filter the existing healthcare data, [2] generate a reference measurement range from the extracted first subset of data, and [3] determine, based on the reference measurement range, measurement unit for lab test data that lack measurement unit or exhibit mislabeling error.

The data integrity maintenance system 100 can include an electronic device 120 and a sensor apparatus 110 that are communicatively coupled to one another (e.g., via one or more wired or wireless communications links 150). In general, the data integrity maintenance system 100 accesses data structures (e.g., health-related data such as existing healthcare data or lab test data stored in a data store, such as database module 122 or otherwise accessible to the electronic device 120, for example, through a server) and determine data integrity (e.g., data quality) of the data structures through processing methods according to implementations described in this disclosure. Further, in some implementations, the data integrity maintenance system 100 obtains sensor data regarding a user using the sensor apparatus 110 and processes the sensor data using the electronic device 120 to determine one or more biomarkers representing the user's medical condition.

In general, the electronic device 120 can include any number of devices that are configured to receive, process, and transmit data. Examples of the electronic device 120 include client computing devices (e.g., desktop computers or notebook computers), server computing devices (e.g., server computers or cloud computing systems), mobile computing devices (e.g., cellular phones, smartphones, tablets, personal data assistants, notebook computers with networking capability), wearable computing devices (e.g., smart phones or headsets), and other computing devices capable of receiving, processing, and transmitting data. In some implementations, the electronic device 120 can include computing devices that operate using one or more operating systems (e.g., Microsoft Windows, Apple macOS, Linux, Unix, Google Android, and Apple iOS, among others) and one or more architectures (e.g., x86, PowerPC, and ARM, among others).

The sensor apparatus 110 includes one or more sensors 112 configured to obtain measurements regarding a physiology of the user, a behavior of the user, and/or any other characteristics of the user. For instance, the sensor apparatus 110 can include, or correspond to, a wearable device (e.g., smart watch), a smart phone, a medical monitoring system, a lab equipment, and more. As an example, the sensor apparatus can include one or more sensors 112 configured to obtain physiological parameters, including vital signs such as glucose level, heart rate, blood pressure, respiratory rate, temperature, or the like. For instance, one or more sensors can be an optical sensor (e.g., PPG), a pulse pressure sensor (PP), a pressure sensor, an electrocardiogram (ECG), bio impedance sensors, galvanic skin response sensors, tonometry/contact sensors, accelerometers, gyroscopes, pressure sensors, acoustic sensors, electro-mechanical movement sensors, and/or electromagnetic sensors. Further, for instance, when the sensor apparatus takes a form of the lab equipment, it can also measure the physiological parameters or perform blood tests, such as analyzing blood glucose levels, cholesterol, and other biomarkers.

Further, the sensor apparatus 110 includes a communications module 116 configured to transmit data and/or receive data from the electronic device 120. As an example, the communications module 116 can include one or more receivers, transmitters, and/or transceivers. In some implementations, the communications module 116 can communicate with the electronic device 120 via one or more wireless links (e.g., serial links, Ethernet links, etc.) and/or wireless links (e.g., Wi-Fi links, Bluetooth links, etc.).

In general, the electronic device 120 is configured to receive sensor data (e.g., physiological parameter data such as clinical parameter(s)) obtained by the sensor apparatus 110, and process the sensor data to determine one or more biomarkers representing the user's medical condition. Further, the electronic device 120 is configured to present information regarding the biomarkers and any other information to the user and/or another user (e.g., a health care provider).

In FIG. 1, the electronic device 120 is illustrated as a single component. However, in practice, the electronic device 120 can be implemented on one or more computing devices (e.g., each computing device including at least one processor such as a microprocessor or microcontroller). As an example, the electronic device 120 can be a single computing device, such as a single smartphone. As another example, the electronic device 120 can include multiple computing devices that are connected via a network (e.g., the Internet, local area network etc.), and the components of the electronic device 120 can be maintained and operated on some or all of the computing devices. For instance, electronic device 120 can include several computing devices, and the components of the electronic device 120 can be distributed on one or more of these computing devices.

Moreover, the electronic device 120 is illustrated as a component that is separate component from the sensor apparatus 110. However, while the electronic device 120 can be a separate component from the sensor apparatus 110, the electronic device 120 can also include, be coupled with, or be adjacent to (e.g., in a housing) the sensor apparatus 110. For example, the electronic device 120 can be a wearable device that includes, is coupled with, or is adjacent to the sensor apparatus 110.

As shown in FIG. 1, the electronic device 120 includes a database module 122, a communications module 124, a processing module 126, and a user interface module 128. The operation modules can be provided as one or more computer executable software modules, hardware modules, or a combination thereof. For example, one or more of the operation modules can be implemented as blocks of software code with instructions that cause one or more processors to execute operations described herein. In addition, or alternatively, one or more of the operations modules can be implemented in electronic circuitry such as, e.g., programmable logic circuits, field programmable logic arrays (FPGA), or application specific integrated circuits (ASIC).

The communications module 124 is configured to transmit data and/or receive data from the sensor apparatus 110. As an example, the communications module 124 can include one or more receivers, transmitters, and/or transceivers. In some implementations, the communications module 124 can communicate with the sensor apparatus 110 (e.g., via the communication module 116) via one or more wired links (e.g., serial links, Ethernet links, etc.) and/or wireless links (e.g., Wi-Fi links, Bluetooth links, etc.).

The database module 122 maintains information related to the operation of the data integrity maintenance system 100.

As an example, the database module 122 can store input data 122a that is used as an input for determining one or more biomarkers representing a health of a user. For instance, the input data 122a can include at least some of the sensor data generated by the sensor apparatus 110.

As another example, the database module 122 can store output data 122b generated by electronic device 120. As an example, the output data 122b can include one or more metrics or biomarkers generated by the electronic device 120 based on the input data 122a.

Further, the database module 122 can store processing rules 122c specifying how data in the database module 122 can be processed to perform the operations described herein.

As an example, the processing rules 122c can include one or more rules that specify how the input data 122a is formatted, parsed, and processed to determine one or more corresponding metrics or biomarkers regarding a user.

As another example, the processing rules 122c can include one or more rules that specify the conditions in which data is presented to a user (e.g., using the user interface module 128), and the manner in which the data is presented.

As another example, the processing rules 122c can include one or more rules that specify the manner in which data is stored for future retrieval and/or processing (e.g., using the database module 122).

Example data processing techniques are described in further detail below.

The processing module 126 processes data stored or otherwise accessible to the electronic device 120. For instance, the processing module 126 can be used to execute one or more of the operations described herein (e.g., by executing the processing rules 122c with respect to the input data 112a in order to generate the output data 122b).

The user interface module 128 is configured to present information to a user and/or to receive inputs from a user. As an example, the user interface module 128 can include one more display devices (e.g., display screens, touch screens, etc.) that are configured to present a user interface (e.g., graphical user interface, GUI) that enables users to interact with the electronic device 120 and/or the sensor apparatus 110. Example interactions include viewing data, transmitting data from one component to another, and/or issuing commands to the electronic device 120 and/or sensor apparatus 110. Commands can include, for example, any user instruction to one or more of the electronic device 120 and/or sensor apparatus 110 to perform particular operations or tasks. In some implementations, the user interface module can also present information to a user aurally (e.g., using one or more speakers) and/or via haptic feedback (e.g., using one more haptic generators, such as a vibration generation).

In some implementations, a software application can be used to facilitate performance of the tasks described herein. As an example, an application can be installed on the electronic device 120. Further, a user can interact with the application to input data and/or commands to the electronic device 120, and review data generated by the electronic device 120.

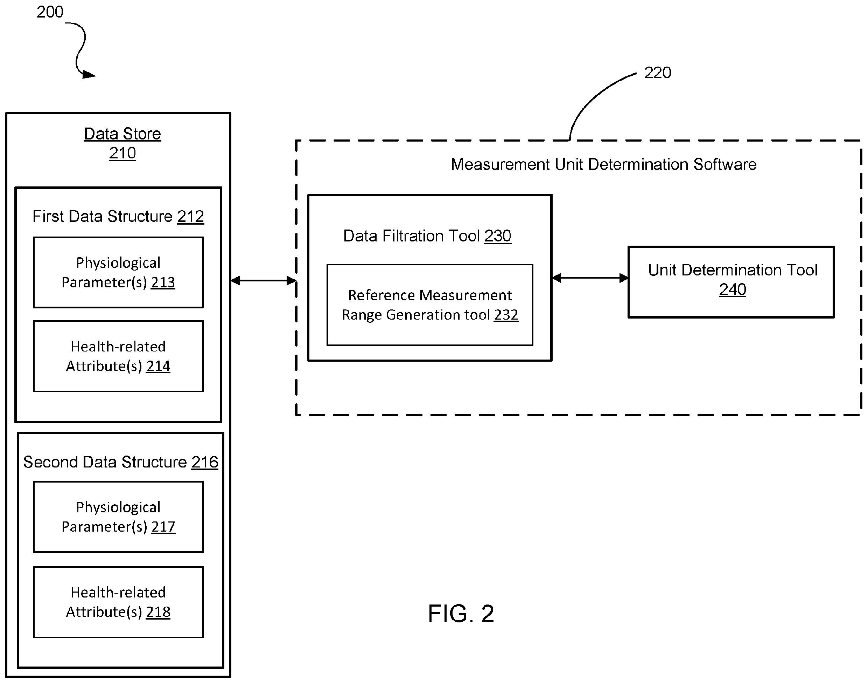

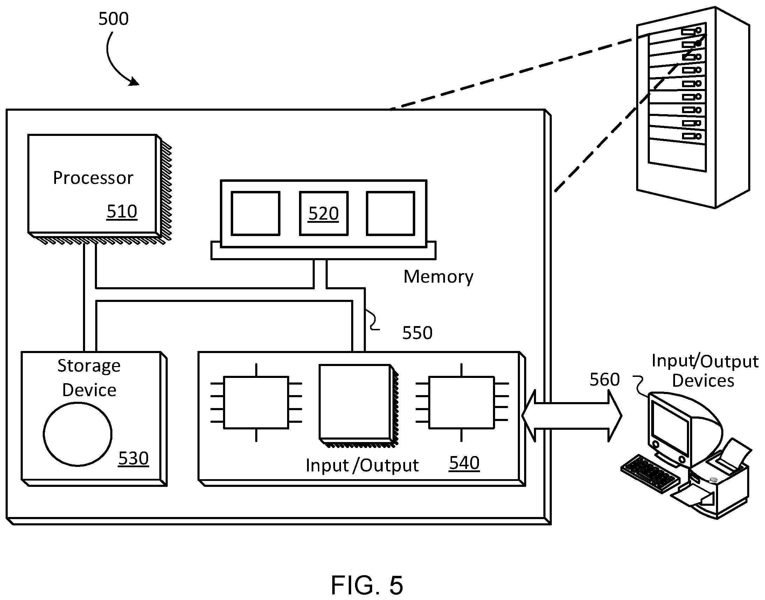

FIG. 2 is an example implementation 200 of a software or algorithm that is utilized by a processor-based electronic device (e.g., the electronic device 120 of the data integrity maintenance system 100 of FIG. 1, a computing device (which can also be a server) of a system 500 of FIG. 5)). In particular, the software or algorithm is utilized by the electronic device to thereby [1] filter the existing healthcare data, [2] generate a reference measurement range from the extracted first subset of data, and [3] determine, based on the reference measurement range, measurement unit for lab test data that lack measurement unit or exhibit mislabeling error.

The example implementation 200 illustrates a data store 210 and a measurement unit determination software 220.

The data store 210 can include, or correspond to, a data store of the electronic device (which can also be a server). For instance, the data store 210 can be the database module 122 of the electronic device 120 and one or more storage devices 530 of the computing device (which can also be a server) of the system 500. The data store 210 can be in data communication with the electronic device (which can also be a server).

The data store 210 can include one or more of first data structure 212 and one or more of second data structure 216. The first data structure 212 can include, or correspond to, existing healthcare data (e.g., historical data, prior clinical trial data, real-time data, and other existing healthcare data). For instance, such existing healthcare data can be used by the electronic device to generate the reference measurement range that is used for [1] determining whether the second data sets of a second data structure 216 (e.g., lab test data) lack measurement unit or exhibit mislabeling error and [2] determining the correct measurement unit for the corresponding lab test data. For instance, the first data structure 212 can include first data sets regarding entities (e.g., individuals, users, patients, subjects, or the like). Each of the first data sets represents measurements of one or more physiological parameters 213 of the entities and one or more health-related attributes 214 of the entities. For instance, one or more physiological parameters can represent clinical parameters that are continuously collected at regularly spaced intervals. For instance, one or more physiological parameters can represent one or more vital signs, such as glucose level, a heart rate, a blood pressure, a respiratory rate, a temperature, or the like. For instance, the one or more health-related attributes can include at least one of a disease indication, a medical condition other than the disease indication, a same medication usage, a same medical treatment, a gender or the like.

The second data structure 216 can include, or correspond to, lab test data that is subject to evaluation for data integrity (e.g., evaluation of whether measurements lack measurement unit or exhibits mislabeling error). For instance, the second data structure 216 can include second data sets regarding target entities. For instance, each of the second data sets represents measurements of one or more physiological parameters 217 of the target entities and one or more health-related attributes 218 of the target entities.

In some implementations, the first data structure 212 and the second data structure 216 can be stored in a different data store. For instance, the first data structure 212 can be stored in a separate server (e.g., a computing device (which can also be a server) of the system 500), while the second data structure 216 can be stored in the data store of the electronic device (e.g., assuming that the electronic device does not take the form of server for this example).

Further, at least some of data filtration or unit detection can be implemented as respective software programs that may be executed the electronic device. A software program can include machine-readable instructions that may be stored in a memory (such as the database module 122 of FIG. 1, a memory 520, a storage device(s) 530 of FIG. 5), and that, when executed by the processor, cause the processor-based electronic device to perform the instructions of the software program. As shown, the measurement unit determination software 220 can include a data filtration tool 230 and/or a unit determination tool 240. In some implementations, the measurement unit determination software 220 can include more or fewer tools. In some implementations, some of the tools may be combined, some of the tools may be split into more tools, or a combination thereof. In some implementations, the measurement unit determination software 220 can be run on a server (e.g., a computing device (which can also be a server) of the system 500), or both the electronic device and the server.

In some implementations, the data filtration tool 230 can take a form of a software different from the measurement unit determination software 220 and run on the server, while the unit determination tool 240 can take a form of the measurement unit determination software 220 and run on the electronic device that is in data communication with the server. Further variations with respect to the data filtration tool 230 and the reference measurement range generation tool 232 being a separate software and being run on the electronic device, the server, or combination thereof, are possible.

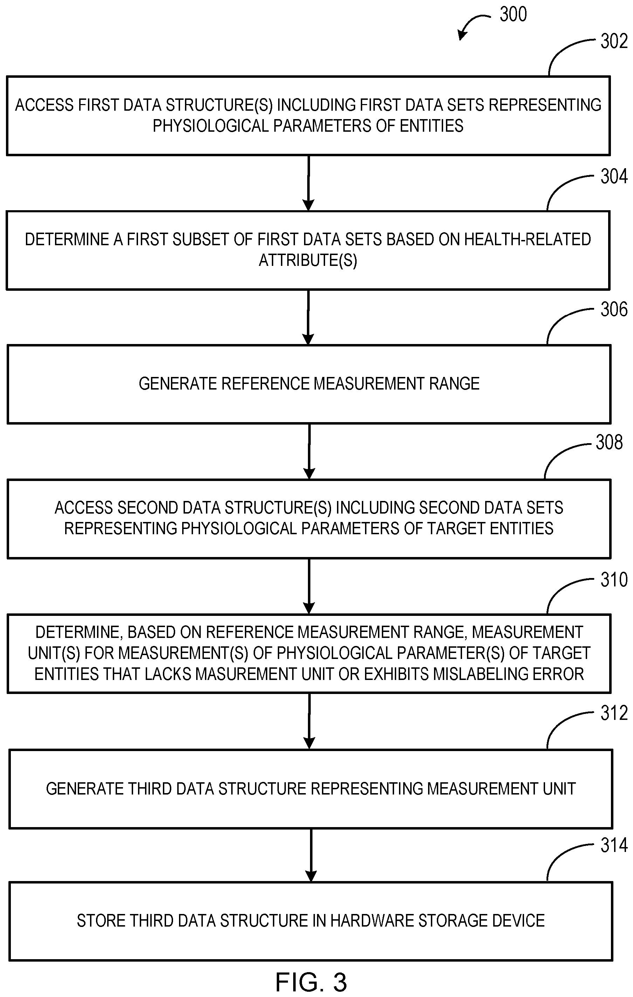

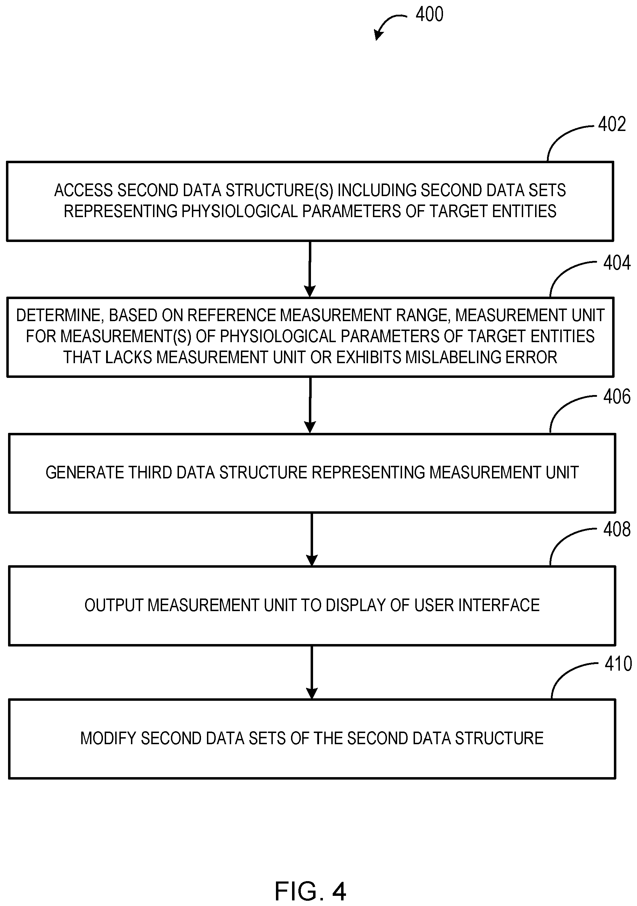

The data filtration tool 230 includes a reference measurement range generation tool 232. For instance, the data filtration tool 230 can first filter such existing healthcare data by at least first determining or extracting a first subset of data based on the one or more health-related attributes 214 which matches the one or more health-related attributes 218 of the target entities. After the first subset of data is determined or extracted based on the health-related attribute(s) 214 and 218, the reference measurement range generation tool 232 can be used to generate the reference measurement range. For instance, generating the reference measurement range includes [1] comparing physiological parameters of the first subset of data with each other and [2] converting different measurement units of the physiological parameters of the first subset to one or more measurement units. More detailed processes are described in example processes 300 and 400 of FIGS. 3 and 4.

The unit determination tool 240 can determine, based on the reference measurement range, measurement unit(s) for the second data sets of the second data structure 216 (e.g., lab data) that lacks measurement unit or exhibits mislabeling error. Moreover, prior to determining the measurement unit, determination of whether second data sets of the second data structure 216 lacks measurement unit or exhibits the mislabeling error can be made. More detailed processes are described in example processes 300 and 400 of FIGS. 3 and 4.

### Example Processes

FIG. 3 is a flow chart diagram of an example process 300 for determining a measurement unit for lab test data. In particular, the example process 300 [1] filters the existing healthcare data (e.g., historical data, prior clinical trial data, real-time data, and other existing healthcare data) by first determining or extracting a first subset of data, such that the first subset of data is focused on common health-related attribute(s), [2] generates a reference measurement range from the first subset of data, and [3] determines, based on the reference measurement range, the measurement unit for the lab test data that lack measurement unit or exhibit mislabeling error. The process 300 can be implemented by a processor-based system, such as the data integrity maintenance system 100 and a system 500, and in conjunction with the example implementation 200, as described in this disclosure.

At 302, one or more first data structures including first data sets is accessed. For instance, a processor-based electronic device (e.g., the electronic device 120 of the data integrity maintenance system 100 or a computing device of system 500) can be used to access the one or more first data structures. For instance, one or more first data structures can correspond to existing healthcare data (e.g., historical data, prior clinical trial data, real-time data, other existing healthcare data, etc.) that is stored in a data store (e.g., the data store 210). For instance, the data store can correspond to the database module 122 of the electronic device 120 or a storage device of computing devices (e.g., one or more storage devices 530 of the computing devices of FIG. 5 that include server). The one or more first data structures can include a plurality of first data sets regarding a plurality of entities (e.g., individuals, users, patients, subjects, or the like) and one or more health-related attributes of the plurality of entities. The first data sets can represent measurement values of one or more physiological parameters of the plurality of entities. For instance, one or more physiological parameters can represent one or more vital signs, such as glucose level, a heart rate, a blood pressure, a respiratory rate, a temperature, or the like.

At 304, a first subset of the first data sets is determined based on one or more health-related attributes. For instance, the electronic device can extract or determine, based on one or more health-related attributes, the first subset of the first data sets. For instance, the one or more health-related attributes can include at least one of a disease indication, a medical condition other than the disease indication, a same medication usage, a same medical treatment, a gender or the like. For instance, the first subset can represent measurement values of one or more physiological parameters of plurality of entities that share common health-related attribute(s). For instance, the one or more physiological parameters of the first subset can represent an outcome variable that measure an effect of the same medication or the same medical treatment. For instance, for illustrative purposes, the entities corresponding to the first subset can correspond to a population group that takes or took a same medication or receives or received the same medical treatment. For instance, the plurality of entities of the first subset can correspond to a population group exhibiting same disease indication.

At 306, one or more reference measurement ranges is generated. For instance, the electronic device can generate the one or more reference measurement ranges by comparing the values (e.g., measurement values) of one or more physiological parameters of the first subset of the first data sets with each other and converting different measurement units of the one or more physiological parameters of the first subset to the one or more measurement units. For instance, for illustrative purposes, when the physiological parameter corresponds to a blood glucose level, and the health-related attribute is certain medication that patients (corresponding to the first subset) take, then within the first subset, there can be many different values (e.g., measurement values) with many different measurement units such as mmol/L (millimoles per liter) or mg/dL (milligrams per deciliter). The electronic device can convert (or unify) those different values with different measurement units into one or more single measurement unit and generate the reference measurement range. For instance, for the blood glucose level, all of the values can be unified into mmol/L or mg/dL and the reference measurement range can be generated in mmol/L, mg/dL, or both.

At 308, one or more second data structures is accessed. For instance, the electronic device can be used to access the one or more second data structures. For instance, the one or more second data structures include one or more second data sets regarding one or more target entities (e.g., target patients, target users, target subjects, or the like). For instance, one or more second data sets can correspond to lab data or target healthcare data that is subject to evaluation for data integrity.

For instance, the one or more second data sets represent measurement values of one or more physiological parameters of the target entities who exhibit or share the one or more health-related attributes that is the same as the health-related attribute(s) of the first subset of the first data sets, from where the reference measurement range is generated. For instance, for illustrative purposes, the target entities corresponding to the one or more second data sets can correspond to a population group that takes or took a same medication or receives or received a same medical treatment. For instance, the one or more target entities' physiological parameter can correspond to a blood glucose level, and the health-related attribute can be a certain medication that the target entities take (in which the certain medication is the same medication that the entities of the first subset of the first data sets take or had taken).

For instance, the one or more second data sets regarding one or more target entities can be subject to evaluation for data integrity. For instance, measurement value(s) of the physiological parameter of the target entities can be missing or exhibit mislabeling error, and the corresponding data (the one or more second data sets such as lab data) can be subject to evaluation for data integrity. For instance, determination of whether measurement value(s) is missing or there is mislabeling error can be made as part of measurement unit(s) determination step at 310.

At 310, measurement unit(s) for one or more measurements of physiological parameters of target entities that lacks measurement unit or exhibits mislabeling error is determined. For instance, the measurement unit(s) can be determined based on the reference measurement range.

For instance, determining the measurement unit(s) can include, [1] first comparing one or more values of the one or more measurements of physiological parameters of target entities to the reference measurement range, [2] determining, based on the comparison, that the one or more measurements exhibits the mislabeling error or lacks the measurement unit, and [3] determining appropriate measurement unit for the one or more measurements.

In some implementations, a score can be generated based on the comparison of the one or more measurement values to the reference measurement range. Thereafter, such score can be compared to a threshold score to determine that the one or more measurements lacks measurement unit or exhibits the mislabeling error. For instance, measurement values of the target entities of the lab test data can be compared against the reference measurement range of each measurement unit, and each comparison (to reference measurement range in each reference unit) can generate a score. Once the score is generated, the score can be compared to a threshold score, and the electronic device can determine whether the one or more measurement values lacks measurement unit or exhibits the mislabeling error.

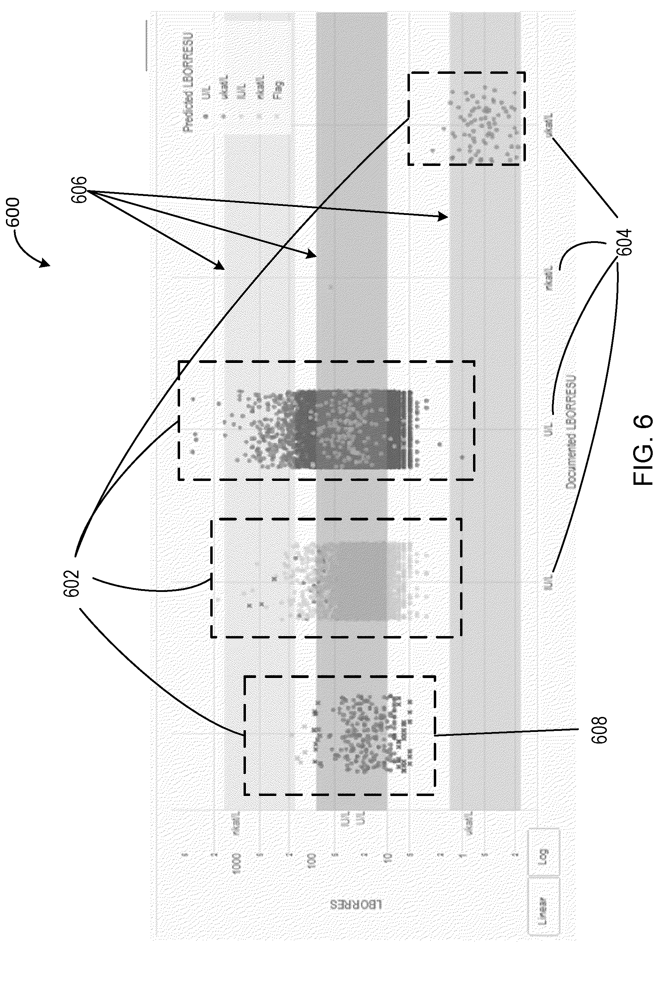

In some implementations, determining that the one or more measurements exhibits the mislabeling error can include determining a frequency of the one or more values of the measurement(s) that falls within the reference measurement range, and determining the mislabeling error based on the frequency. For instance, for illustrative purposes, when the physiological parameter is blood glucose level, and the reference range is 70-100 mg/dL, then the blood glucose values of the target entities can form a frequency cluster (e.g., as depicted by frequency of one or more measurements 602 in FIG. 6) within this reference range or within the threshold outside this reference range. As such, values that fall outside the reference range or the threshold (e.g., ± 20% of the maximum value or minimum value of the reference range) can be deemed as data points that exhibit mislabeling error.

In some implementations, machine learning classifier can be used to determine the measurement unit for the one or more measurements that lacks the measurement unit or exhibits the mislabeling error regarding the measurement unit. For instance, the machine learning classifier can be trained based on z-score probability and one or more of frequency features that are extracted from the one or more first data structures (e.g., existing healthcare data such as historical data), where the frequency features include a unit frequency by study, a unit frequency by site, and a unit frequency by subject. For instance, the frequency features are described below in Table 1. For instance, logistic regression model can be used to predict, based on the z-score probability derived from the reference measurement range and the frequency features, a binary outcome that indicates a possibility of the one or more measurements being measured in one or more respective measurement units. For instance, the probability outcome from the classifier can act as another threshold for detecting mislabeled units which helps decrease the false positive rate.

**Table 1: Features used in machine learning classifier for unit determination or prediction**

| Features | Description |
|---|---|
| z-score probability | Inferred probability for the value to be in the reference measurement range of labeled unit |
| Unit frequency by study | Proportion of the records labeled with the same unit within the same study, range = [0,1] |
| Unit frequency by site | Proportion of the records labeled with the same unit within the same site, range = [0,1] |
| Unit frequency by subject | Proportion of the records labeled with the same unit within the same subject, range = [0,1] |

At 312, third data structure representing the determined measurement unit is generated. For instance, the electronic device can format the data into a standardized data format, such as JavaScript Object Notation (JSON) or Extensible Markup Language (XML) format. The standardized data format can be other appropriate data format suitable for storage in a data store or a hardware storage device.

At 314, the third data structure is stored in the hardware storage device. For example, the data structure can be stored in the data store (e.g., the data store 210, the database module 122 of the electronic device 120, the one or more storage devices 530, etc.).

In some implementations, in supplant of, or in addition to, storing the data structure in the hardware storage device, the data structure may be output to a user interface (e.g. using the user interface module 128). For example, in cases of outputting the data structure for user display without storing the data structure, the data structure may be generated in other format appropriate for display at step 312. For example, in cases of outputting the data structure for user display after storing the data structure, the data structure can be converted from the storage format to the appropriate display format, and be output.

In some implementations, the third data structure can be provided or transmitted to a computerized health analysis platform.

FIG. 4 is a flow chart diagram of an example process 400 for determining a measurement unit for lab test data and modifying the lab test data. In particular, the example process 400 [1] determines measurement unit for lab test data that lack measurement unit or exhibit mislabeling error and [2] modifies the one or more second data sets (of the second data structure). The process 400 can be implemented by a processor-based system, such as the data integrity maintenance system 100 and a system 500, and in conjunction with the example implementation 200 and the example process 300, as described in this disclosure.

At 402, one or more second data structures is accessed. As the technique used in step 402 can be the same as the technique used in step 308 of FIG. 3, the technique is not repeated here.

At 404, measurement unit(s) for one or more measurements of physiological parameters of target entities that lacks measurement unit or exhibits mislabeling error is determined. For instance, the measurement unit(s) can be determined based on the reference measurement range. As the technique used in step 404 can be the same as the technique used in step 310 of FIG. 3, the technique is not repeated here.

At 406, third data structure representing the determined measurement unit is generated. As the technique used in step 406 can be the same as the technique used in step 312 of FIG. 3, the technique is not repeated here.

At 408, the measurement unit is output for display on user interface (e.g. using the user interface module 128). For instance, a graph representing the first subset of the time-series data can be generated for display on the user interface. For instance, an indication of whether one or more measurements exhibits the mislabeling error or lacks the measurement unit is generated on the display of the user interface. For instance, a frequency graph that represents a relationship between a frequency of the one or more measurements and a plurality of measurement units is generated on the display of the user interface.

For instance, FIG. 6 is a frequency graph 600 that illustrates a relationship between a frequency of one or more measurements 602 and the plurality of measurement units 604. For instance, x-axis depicts the plurality of measurement units 605 and y-axis depicts actual measurement values of physiological parameter. For instance, shaded regions 606 represent multiple reference measurement ranges in multiple units. Moreover, for instance, first column 608 illustrates a set of points or measurement values which lack measurement unit. For instance, implementations described in this disclosure, including the process 300 and the process 400, have determined what the measurement unit should be for these measurement values in the first column 608 that lack measurement unit, based on the reference measurement range of each measurement unit as shown by a respective shaded region of the shaded regions 606. On the other hand, when the data points [1] are not within a main shaded region where the most data points belong to (e.g., region with the highest frequency), [2] but rather fall into different shaded regions within the same measurement unit category on the x-axis, then these data points can exhibit mislabeling error. In such cases, for example, a measurement unit corresponding to the different shaded region that captures these data points (that are outside the main shaded region) can be the appropriate measurement unit for those data points.

In some implementations, when one or more measurements exhibits the mislabeling error or lacks the measurement unit, data points corresponding to the one or more measurements can be subject to review and be flagged as records or data points that require review or verification.

At 410, the one or more second data sets of the second data structure is modified. For instance, the second data sets can be modified based on the determined measurement unit. For instance, modifying includes [1] incorporating the determined measurement unit to the one or more measurements that lacks the measurement unit or [2] replacing respective measurement unit of the one or more measurements that exhibits the mislabeling error with the determined measurement unit. By this modification, data quality of the one or more second data sets can be improved by preventing processing of incomplete or erroneous second data sets. In some implementations, such modification can be made after the flagged records or data points that require review or verification has been reviewed or verified.

In some implementations, modified second data sets or the second data structure can be provided or transmitted to a computerized health analysis platform.

### Example Computer Systems

FIG. 5 depicts an example computing system, according to implementations of the present disclosure. The system 500 may be used for any of the operations described with respect to the various implementations discussed herein. The system 500 may be included in, used by, in communication with, or correspond to the electronic device 120. Further, the system 500 may include, used by, or in communication with the sensor apparatus 110. The system 500 may include one or more processors 510, a memory 520, one or more storage devices 530, and one or more input/output (I/O) devices 560 controllable through one or more I/O interfaces 540. The various components 510, 520, 530, 540, or 560 may be interconnected through at least one system bus 550, which may enable the transfer of data between the various modules and components of the system 500.

The processor(s) 510 may be configured to process instructions for execution within the system 500. The processor(s) 510 may include single-threaded processor(s), multi-threaded processor(s), or both. The processor(s) 510 may be configured to process instructions stored in the memory 520 or on the storage device(s) 530. The processor(s) 510 may include hardware-based processor(s) each including one or more cores. The processor(s) 510 may include general purpose processor(s), special purpose processor(s), or both.

The memory 520 may store information within the system 500. In some implementations, the memory 520 includes one or more computer-readable media. The memory 520 may include any number of volatile memory units, any number of non-volatile memory units, or both volatile and non-volatile memory units. The memory 520 may include read-only memory, random access memory, or both. In some examples, the memory 520 may be employed as active or physical memory by one or more executing software modules.

The storage device(s) 530 may be configured to provide (e.g., persistent) mass storage for the system 500. In some implementations, the storage device(s) 530 may include one or more computer-readable media. For example, the storage device(s) 530 may include a floppy disk device, a hard disk device, an optical disk device, or a tape device. The storage device(s) 530 may include read-only memory, random access memory, or both. The storage device(s) 530 may include one or more of an internal hard drive, an external hard drive, or a removable drive.

One or both of the memory 520 or the storage device(s) 530 may include one or more computer-readable storage media (CRSM). The CRSM may include one or more of an electronic storage medium, a magnetic storage medium, an optical storage medium, a magneto-optical storage medium, a quantum storage medium, a mechanical computer storage medium, and so forth. The CRSM may provide storage of computer-readable instructions describing data structures, processes, applications, programs, other modules, or other data for the operation of the system 500. In some implementations, the CRSM may include a data store that provides storage of computer-readable instructions or other information in a non-transitory format. The CRSM may be incorporated into the system 500 or may be external with respect to the system 500. The CRSM may include read-only memory, random access memory, or both. One or more CRSM suitable for tangibly embodying computer program instructions and data may include any type of non-volatile memory, including but not limited to: semiconductor memory devices, such as EPROM, EEPROM, and flash memory devices; magnetic disks such as internal hard disks and removable disks; magneto-optical disks; and CD-ROM and DVD-ROM disks. In some examples, the processor(s) 510 and the memory 520 may be supplemented by, or incorporated into, one or more application-specific integrated circuits (ASICs).

The system 500 may include one or more I/O devices 560. The I/O device(s) 560 may include one or more input devices such as a keyboard, a mouse, a pen, a game controller, a touch input device, an audio input device (e.g., a microphone), a gestural input device, a haptic input device, an image or video capture device (e.g., a camera), or other devices. In some examples, the I/O device(s) 560 may also include one or more output devices such as a display, LED(s), an audio output device (e.g., a speaker), a printer, a haptic output device, and so forth. The I/O device(s) 560 may be physically incorporated in one or more computing devices of the system 500, or may be external with respect to one or more computing devices of the system 500.

The system 500 may include one or more I/O interfaces 540 to enable components or modules of the system 500 to control, interface with, or otherwise communicate with the I/O device(s) 560. The I/O interface(s) 540 may enable information to be transferred in or out of the system 500, or between components of the system 500, through serial communication, parallel communication, or other types of communication. For example, the I/O interface(s) 540 may comply with a version of the RS-232 standard for serial ports, or with a version of the IEEE 1284 standard for parallel ports. As another example, the I/O interface(s) 540 may be configured to provide a connection over Universal Serial Bus (USB) or Ethernet. In some examples, the I/O interface(s) 540 may be configured to provide a serial connection that is compliant with a version of the IEEE 1394 standard.

The I/O interface(s) 540 may also include one or more network interfaces that enable communications between computing devices in the system 500, or between the system 500 and other network-connected computing systems. The network interface(s) may include one or more network interface controllers (NICs) or other types of transceiver devices configured to send and receive communications over one or more networks using any network protocol.

Computing devices of the system 500 may communicate with one another, or with other computing devices, using one or more networks. Such networks may include public networks such as the internet, private networks such as an institutional or personal intranet, or any combination of private and public networks. The networks may include any type of wired or wireless network, including but not limited to local area networks (LANs), wide area networks (WANs), wireless WANs (WWANs), wireless LANs (WLANs), mobile communications networks (e.g., 3G, 4G, Edge, etc.), and so forth. In some implementations, the communications between computing devices may be encrypted or otherwise secured. For example, communications may employ one or more public or private cryptographic keys, ciphers, digital certificates, or other credentials supported by a security protocol, such as any version of the Secure Sockets Layer (SSL) or the Transport Layer Security (TLS) protocol.

The system 500 may include any number of computing devices of any type. The computing device(s) may include, but are not limited to: a personal computer, a smartphone, a tablet computer, a wearable computer, an implanted computer, a mobile gaming device, an electronic book reader, an automotive computer, a desktop computer, a laptop computer, a notebook computer, a game console, a home entertainment device, a network computer, a server computer, a mainframe computer, a distributed computing device (e.g., a cloud computing device), a microcomputer, a system on a chip (SoC), a system in a package (SiP), and so forth. Although examples herein may describe computing device(s) as physical device(s), implementations are not so limited. In some examples, a computing device may include one or more of a virtual computing environment, a hypervisor, an emulation, or a virtual machine executing on one or more physical computing devices. In some examples, two or more computing devices may include a cluster, cloud, farm, or other grouping of multiple devices that coordinate operations to provide load balancing, failover support, parallel processing capabilities, shared storage resources, shared networking capabilities, or other aspects.

This specification uses the term "configured" in connection with systems and computer program components. For a system of one or more computers to be configured to perform particular operations or actions means that the system has installed on software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively, or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term "database" is used broadly to refer to any collection of data: the data does not need to be structured in any particular way, or structured at all, and it can be stored on storage devices in one or more locations. Thus, for example, the index database can include multiple collections of data, each of which may be organized and accessed differently.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks.

The term "memory subsystem" can include one or more memories, where each memory may be a computer-readable medium. A memory subsystem may encompass memory hardware units (e.g., a hard drive or a disk) that store data or instructions in software form. Alternatively or in addition, the memory subsystem may include data or instructions that are hard-wired into processing circuitry.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable sub combination. Moreover, although features may be described above as acting in certain combinations and even initially be claimed as such, one or more features from a claimed combination can in some cases be excised from the combination, and the claimed combination may be directed to a sub combination or variation of a sub combination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

### EMBODIMENTS

Although the present invention is defined in the claims, it should be understood that the present invention can also (alternatively) be defined in accordance with the following embodiments:
1. A system for maintaining data integrity in a computerized health analysis platform, the system comprising:
   at least one processor; and
   a memory subsystem communicatively coupled to the at least one processor, the memory subsystem storing instructions which, when executed by the at least one processor, cause the at least one processor to perform operations comprising:
      accessing one or more first data structures comprising:
         a plurality of first data sets regarding a plurality of entities, wherein each of the first data sets represents measurements of one or more physiological parameters of the plurality of entities, wherein a machine transmitting the data structures determines that the system accessing them is authorized to access them , and
         one or more health-related attributes of the plurality of entities;
      determining a first subset of the first data sets based on the one or more health-related attributes of the plurality of entities;
      generating, in one or more measurement units, a reference measurement range of the one or more physiological parameters of plurality of entities of the first subset of the first data sets;
      accessing one or more second data structures comprising:
         one or more second data sets regarding one or more target entities, wherein the one or more second data sets represent one or
         more measurements of the one or more physiological parameters of the one or more target entities,
            wherein the one or more target entities exhibit the one or more health-related attributes, and
            wherein the one or more measurements of the one or more target entities lack a measurement unit or exhibit a mislabeling error regarding the measurement unit;
         determining, based on the reference measurement range, the measurement unit for the one or more measurements that lacks the measurement unit or exhibits the mislabeling error regarding the measurement unit;
            generating a third data structure representing the measurement unit; and storing the third data structure in a hardware storage device.
2. The system of embodiment 1, wherein the operations further comprise providing the third data structure to the computerized health analysis platform.
3. The system of any one of embodiments 1 or 2, wherein the one or more health-related attributes comprise at least one of a disease indication, a medical condition other than the disease indication, a same medication usage, a same medical treatment, or a gender.
4. The system of any one of embodiments 1 to 3, wherein the one or more physiological parameters represent clinical parameters that are continuously collected at regularly spaced intervals.
5. The system of any one of embodiments 1 to 4, wherein the one or more physiological parameters represents one or more vital signs.
6. The system of embodiment 5, wherein the one or more vital signs comprise at least one of a glucose level, a heart rate, a blood pressure, a respiratory rate, or a temperature.
7. The system of any one of embodiments 1 to 6, wherein:
   the plurality of entities of the first subset corresponds to a population group exhibiting same disease indication.
8. The system of any one of embodiments 1 to 7, wherein:
   the plurality of entities of the first subset corresponds to a population group that takes a same medication or receives a same medical treatment; and
   the one or more physiological parameters of the first subset represents an outcome variable that measure an effect of the same medication or the same medical treatment.
9. The system of any one of embodiments 1 to 8, wherein generating the reference measurement range comprises:
   comparing measurements values of the one or more physiological parameters of the first subset of the first data sets with each other; and
   converting different measurement units of the one or more physiological parameters of the first subset to the one or more measurement units.
10. The system of embodiment 9, wherein determining the measurement unit for the one or more measurements that lacks the measurement unit or exhibits the mislabeling error regarding the measurement unit comprises:
   comparing one or more values of the one or more measurements to the reference measurement range; and
   determining, based on the comparison, that the one or more measurements exhibits the mislabeling error or lacks the measurement unit.
11. The system of embodiment 10, wherein determining that the one or more measurements exhibits the mislabeling error comprises:
   determining a frequency of the one or more values of the one or more measurements that falls within the reference measurement range, and
   determining the mislabeling error based on the frequency.
12. The system of any one of embodiments 10 or 11, wherein determining the measurement unit comprises:
   generating a score based on the comparison of the one or more values of the one or more measurements to the reference measurement range.
13. The system of embodiment 12, wherein the one or more measurements exhibits the mislabeling error, and
   wherein determining the measurement unit comprises:
   based on comparing the score to a threshold score, determining the one or more measurements exhibits the mislabeling error.
14. The system of any one of embodiments 9 to 13, wherein the operations further comprise:
   outputting the measurement unit to a display of a user interface.
15. The system of embodiment 14, wherein the operations further comprise:
   generating, on the display of the user interface, an indication of whether one or more measurements exhibits the mislabeling error or lacks the measurement unit.
16. The system of embodiment 15, wherein the operations further comprise:
   generating a frequency graph that represents a relationship between a frequency of the one or more measurements and a plurality of measurement units.
17. The system of any one of embodiments 1 to 16, wherein determining the measurement unit for the one or more measurements that lacks the measurement unit or exhibits the mislabeling error regarding the measurement unit comprises:
   utilizing a machine learning classifier to determine the measurement unit,
   wherein the machine learning classifier is trained based on z-score probability and one or more of frequency features, and
   wherein the frequency features comprise a unit frequency by study, a unit frequency by site, and a unit frequency by subject.
18. The system of embodiment 17, wherein utilizing a machine learning classifier to determine the measurement unit comprises:
   utilizing logistic regression model to predict, based on the z-score probability derived from the reference measurement range and the frequency features, a binary outcome that indicates a possibility of the one or more measurements being measured in one or more respective measurement units.
19. The system of any one of embodiments 1 to 18, wherein the operations further comprise:
   modifying, based on the third data structure, the one or more second data sets.
20. The system of embodiment 19, wherein modifying the one or more second data sets improves data integrity by preventing processing of incomplete or erroneous second data sets, wherein the measurement unit (i) is incorporated into the one or more measurements that lacks the measurement unit or (ii) replaces respective measurement unit of the one or more measurements that exhibits the mislabeling error.
21. A method comprising:
   accessing, by an electronic device, one or more first data structures comprising:
      a plurality of first data sets regarding a plurality of entities, wherein each of the first data sets represents measurements of one or more physiological parameters of the plurality of entities, and
      one or more health-related attributes of the plurality of entities;
   determining, by the electronic device, a first subset of the first data sets based on the one or more health-related attributes of the plurality of entities;
   generating, by the electronic device and in one or more measurement units, a reference measurement range of the physiological parameters of plurality of entities of the first subset of the first data sets;
   accessing, by the electronic device, one or more second data structures comprising:
      one or more second data sets regarding one or more target entities,
      wherein the one or more second data sets represents one or more measurements of the one or more physiological parameters of the one or more target entities,
      wherein the one or more target entities exhibits the one or more health-related attributes, and
      wherein the one or more measurements lacks a measurement unit or exhibits a mislabeling error regarding the measurement unit;
   determining, by the electronic device, the measurement unit for the one or more measurements that lacks the measurement unit or exhibits the mislabeling error regarding the measurement unit;
   generating, by the electronic device, a third data structure representing the measurement unit; and
   storing, by the electronic device, the third data structure in a hardware storage device.
22. The method of embodiment 21, further comprising:
   providing, by the electronic device, the third data structure to a computerized health analysis platform.
23. The method of any one of embodiments 21 or 22, wherein the one or more health-related attributes comprise at least one of a disease indication, a medical condition other than the disease indication, a same medication usage, a same medical treatment, or a gender.
24. The method of any one of embodiments 21 to 23, wherein the one or more physiological parameters represents one or more vital signs.
25. The method of any one of embodiments 21 to 24, wherein:
   the plurality of entities of the first subset corresponds to a population group that takes a same medication or receive a same medical treatment; and
   the one or more physiological parameters of the first subset represents an outcome variable that measure an effect of the same medication or the same medical treatment.
26. The method of any one of embodiments 21 to 25, wherein generating the reference measurement range comprises:
   comparing measurements values of the one or more physiological parameters of the first subset of the first data sets with each other; and
   converting different measurement units of the one or more physiological parameters of the first subset to the one or more measurement units.
27. The method of any one of embodiments 21 to 26, wherein determining the measurement unit for the one or more measurements that lacks the measurement unit or exhibits the mislabeling error regarding the measurement unit comprises:
   comparing one or more values of the one or more measurements to the reference measurement range; and
   determining, based on the comparison, that the one or more measurements exhibits the mislabeling error or lacks the measurement unit.
28. The method of embodiment 27, wherein determining that the one or more measurements exhibits the mislabeling error comprises:
   determining a frequency of the one or more measurements that falls within the reference measurement range,
   determining the mislabeling error based on the frequency.
29. The method of any one of embodiments 27 or 28, wherein determining the measurement unit comprises:
   generating a score based on the comparison of the one or more values of the one or more measurements to the reference measurement range.
30. The method of embodiment 29, wherein the one or more measurements lacks the measurement unit, and
   wherein determining the measurement unit comprises:
   based on comparing the score to a threshold score, determining that the one or more measurements lacks the measurement unit.
31. The method of embodiment 29, wherein the one or more measurements exhibits the mislabeling error, and
   wherein determining the measurement unit comprises, based on comparing the score to a threshold score, determining the one or more measurements exhibits the mislabeling error.
32. The method of any one of embodiments 27 to 31, further comprising:
   outputting, by the electronic device, the measurement unit to a display of a user interface.
33. The method of embodiment 32, further comprising:
   generating, on the display of the user interface and by the electronic device, an indication of whether one or more measurements exhibits the mislabeling error or lacks the measurement unit.
34. The method of embodiment 33, further comprising:
   generating, by the electronic device, a frequency graph that represents a relationship between a frequency of the one or more measurements and a plurality of measurement units.
35. The method of any one of embodiments 21 to 34, wherein determining the measurement unit for the one or more measurements that lacks the measurement unit or exhibits the mislabeling error regarding the measurement unit comprises:
   utilizing a machine learning classifier to determine the measurement unit,
   wherein the machine learning classifier is trained based on z-score probability and one or more of frequency features, and
   wherein the frequency features comprise a unit frequency by study, a unit frequency by site, and a unit frequency by subject.
36. The method of embodiment 35, wherein utilizing the machine learning classifier to determine the measurement unit comprises:
   utilizing a logistic regression model to predict, based on the z-score probability derived from the reference measurement range and the frequency features, a binary outcome that indicates a possibility of the one or more measurements being measured in one or more respective measurement units.
37. One or more non-transitory computer-readable media storing instructions which, when executed by at least one processor, cause the at least one processor to perform the method of any one of embodiments 21-36.

## Claims

1. A computer-implemented method comprising:
accessing one or more first data structures comprising:
a plurality of first data sets regarding a plurality of entities, wherein each of the first data sets represents measurements of one or more physiological parameters of the plurality of entities, and
one or more health-related attributes of the plurality of entities;
determining a first subset of the first data sets based on the one or more health-related attributes of the plurality of entities;
generating, in one or more measurement units, a reference measurement range of the physiological parameters of plurality of entities of the first subset of the first data sets;
accessing one or more second data structures comprising:
one or more second data sets regarding one or more target entities,
wherein the one or more second data sets represents one or more measurements of the one or more physiological parameters of the one or more target entities,
wherein the one or more target entities exhibits the one or more health-related attributes, and
wherein the one or more measurements lacks a measurement unit or exhibits a mislabeling error regarding the measurement unit;
determining the measurement unit for the one or more measurements that lacks the measurement unit or exhibits the mislabeling error regarding the measurement unit;
generating a third data structure representing the measurement unit; and
storing, by the electronic device, the third data structure in a hardware storage device.

2. The method of claim 1, further comprising:
providing, by the electronic device, the third data structure to a computerized health analysis platform.

3. The method of any one of claims 1 or 2, wherein the one or more health-related attributes comprise at least one of a disease indication, a medical condition other than the disease indication, a same medication usage, a same medical treatment, or a gender; and/or
wherein the one or more physiological parameters represent clinical parameters that are continuously collected at regularly spaced intervals; and/or wherein the one or more physiological parameters represents one or more vital signs, optionally wherein the one or more vital signs comprise at least one of a glucose level, a heart rate, a blood pressure, a respiratory rate, or a temperature; and/or
wherein the plurality of entities of the first subset corresponds to a population group exhibiting same disease indication; and/or
wherein:
the plurality of entities of the first subset corresponds to a population group that takes a same medication or receive a same medical treatment, and
the one or more physiological parameters of the first subset represents an outcome variable that measure an effect of the same medication or the same medical treatment.

4. The method of any one of claims 1 to 3, wherein generating the reference measurement range comprises:
comparing measurements values of the one or more physiological parameters of the first subset of the first data sets with each other; and
converting different measurement units of the one or more physiological parameters of the first subset to the one or more measurement units.

5. The method of any one of claims 1 to 4, wherein determining the measurement unit for the one or more measurements that lacks the measurement unit or exhibits the mislabeling error regarding the measurement unit comprises:
comparing one or more values of the one or more measurements to the reference measurement range; and
determining, based on the comparison, that the one or more measurements exhibits the mislabeling error or lacks the measurement unit.

6. The method of claim 5, wherein determining that the one or more measurements exhibits the mislabeling error comprises:
determining a frequency of the one or more measurements that falls within the reference measurement range,
determining the mislabeling error based on the frequency.

7. The method of claim 5, wherein determining the measurement unit comprises:
generating a score based on the comparison of the one or more values of the one or more measurements to the reference measurement range.

8. The method of claim 7, wherein the one or more measurements lacks the measurement unit, and
wherein determining the measurement unit comprises:
based on comparing the score to a threshold score, determining that the one or more measurements lacks the measurement unit.

9. The method of claim 7, wherein the one or more measurements exhibits the mislabeling error, and
wherein determining the measurement unit comprises, based on comparing the score to a threshold score, determining the one or more measurements exhibits the mislabeling error.

10. The method of claim 5, further comprising:
outputting, by the electronic device, the measurement unit to a display of a user interface.

11. The method of claim 10, further comprising:
generating, on the display of the user interface and by the electronic device, an indication of whether one or more measurements exhibits the mislabeling error or lacks the measurement unit; optionally
wherein the method further comprises:
generating, by the electronic device, a frequency graph that represents a relationship between a frequency of the one or more measurements and a plurality of measurement units.

12. The method of any one of claims 1 to 11, wherein determining the measurement unit for the one or more measurements that lacks the measurement unit or exhibits the mislabeling error regarding the measurement unit comprises:
utilizing a machine learning classifier to determine the measurement unit,
wherein the machine learning classifier is trained based on z-score probability and one or more of frequency features, and
wherein the frequency features comprise a unit frequency by study, a unit frequency by site, and a unit frequency by subject; optionally
wherein utilizing the machine learning classifier to determine the measurement unit comprises:
utilizing a logistic regression model to predict, based on the z-score probability derived from the reference measurement range and the frequency features, a binary outcome that indicates a possibility of the one or more measurements being measured in one or more respective measurement units.

13. The method of any one of claims 1 to 12, wherein the operations further comprise:
modifying, based on the third data structure, the one or more second data sets; optionally
wherein modifying the one or more second data sets improves data integrity by preventing processing of incomplete or erroneous second data sets, wherein the measurement unit (i) is incorporated into the one or more measurements that lacks the measurement unit or (ii) replaces respective measurement unit of the one or more measurements that exhibits the mislabeling error.

14. A system for maintaining data integrity in a computerized health analysis platform, the system comprising:
at least one processor; and
a memory subsystem communicatively coupled to the at least one processor, the memory subsystem storing instructions which, when executed by the at least one processor, cause the at least one processor to perform the method of any one of claims 1 to 13.

15. One or more non-transitory computer-readable media storing instructions which, when executed by at least one processor, cause the at least one processor to perform the method of any of claims 1 to 13.
